# EUROPEAN PATENT SPECIFICATION

(11) **EP 3 115 358 B1**
(45) Date of publication and mention of the grant of the patent: **11.09.2019**
(21) Application number: 14884887.2
(22) Date of filing: 04.03.2014
(51) Int. Cl.: C07D 211/42, C07B 53/00

(54) **METHOD OF PREPARATION AND CHIRAL INVERSION OF CHIRAL-1-T-BUTOXYCARBONYL-3-HYDROXY PIPERIDINE**
VERFAHREN ZUR HERSTELLUNG UND CHIRALE INVERSION VON CHIRALEM -1-T-BUTOXYCARBONYL-3-HYDROXYPIPERIDIN
PROCÉDÉ DE PRÉPARATION ET D'INVERSION CHIRALE DE 1-T-BUTOXYCARBONYL-3-HYDROXY PIPÉRIDINE CHIRALE

(43) Date of publication of application: 11.01.2017
(73) Proprietor: ABA Chemicals Corporation, Taicang, Jiangsu 215433 (CN)
(72) Inventor: LIN, Zhigang, Taicang Jiangsu 215433 (CN); XU, Jun, Taicang Jiangsu 215433 (CN); LIU, Yanqin, Taicang Jiangsu 215433 (CN); QUE, Limin, Taicang Jiangsu 215433 (CN); JIANG, Yueheng, Taicang Jiangsu (CN); CAI, Tong, Taicang Jiangsu (CN)
(74) Representative: Schüssler, Andrea
(86) International application number: PCT/CN2014/000197
(87) International publication number: WO 2015/131299

(56) References cited:
- WO-A1-2012/138678
- WO-A2-2004/064730
- CN-A- 1 738 793
- CN-A- 1 809 576
- CN-A- 103 864 673
- US-A- 4 501 748
- RICHARD LIHAMMAR ET AL: "An Efficient Dynamic Kinetic Resolution of N-Heterocyclic 1,2-Amino Alcohols", ADVANCED SYNTHESIS & CATALYSIS, vol. 353, no. 13, 26 September 2011 (2011-09-26), pages 2321-2327, XP055319451, DE ISSN: 1615-4150, DOI: 10.1002/adsc.201100466
- Reinhard Bruckner: "Stereochemistry of SN2 Substitutions" In: "Advanced Organic Chemistry", 1 January 2001 (2001-01-01), Elsevier, XP055444278, pages 51-52, * the whole document *

## Description

### TECHNICAL FIELD

The present invention relates to the synthesis of a compound, specifically to a method of preparation and chiral inversion of chiral-1-t-butoxycarbonyl-3-hydroxy piperidine.

### BACKGROUND ART

(S)-1-t-butoxycarbonyl-3-hydroxy piperidine is an important pharmaceutical intermediate. Its synthetic method reported in present literature is mainly as follows:
Method one (asymmetric synthesis): 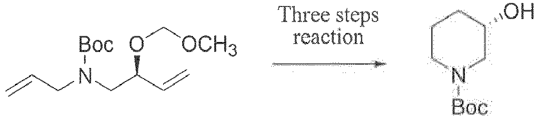 Main literature: Tetrahedron, 63, 331-336; 2007.
Method two (enzymatic asymmetric reduction): 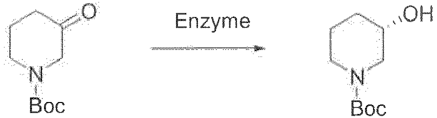 Main literature: Organic Letters, 11(6), 1245-1248; 2009.
Method three (chemical resolution method): 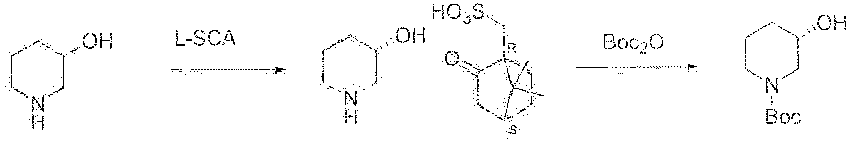 Main literatures: WO2004/064730 A2; WO2004/072086 A2; Lihammar et al., Advanced Synthesis & Catalysis, Vol. 353, No. 13, pp. 2321-2327 (2011).

In the chemical resolution method, there are problems that 3-hydroxy piperidine is not easy to be purified to obtain and hard to be industrialized due to its water solubility. Meanwhile, chiral impurities are easily existed in the resolution process.

### SUMMARY OF THE INVENTION

The object of the present invention is to provide a new preparation method of chiral 1-t-butoxycarbonyl-3-hydroxy piperidine. The synthetic route has not been reported in literature, its reaction conditions are mild, and it is suitable for industrial mass production.

Specifically, the present invention provides a preparation method of chiral 1-t-butoxycarbonyl-3-hydroxy piperidine, which mainly comprises steps of:
1) Using N-benzyl-3-hydroxy piperidine of formula I as raw material, and resolving it under the action of chiral camphorsulfonic acid (CSA) to obtain (S)-1-benzyl-3-hydroxy piperidine camphorsulfonate of formula II s or (R)-1-benzyl-3-hydroxy piperidine camphorsulfonate of formula II r;
2) Dissociating the (S)-1-benzyl-3-hydroxy piperidine camphorsulfonate of formula II s or (R)-1-benzyl-3-hydroxy piperidine camphorsulfonate of formula II r of step 1) by an alkali to obtain (S)-1-benzyl-3-hydroxy piperidine of formula III s or (R)-1-benzyl-3-hydroxy piperidine of formula III r;
3) Catalytic hydrogenating debenzylating the (S)-1-benzyl-3-hydroxy piperidine of formula III s or (R)-1-benzyl-3-hydroxy piperidine of formula III r of step 2) by palladium-charcoal, meanwhile, protecting by t-butoxycarbonyl acid anhydride to obtain (S)-1-t-butoxycarbonyl-3-hydroxy piperidine of formula IV s or (R)-1-butoxycarbonyl-3-hydroxy piperidine of formula IV r.

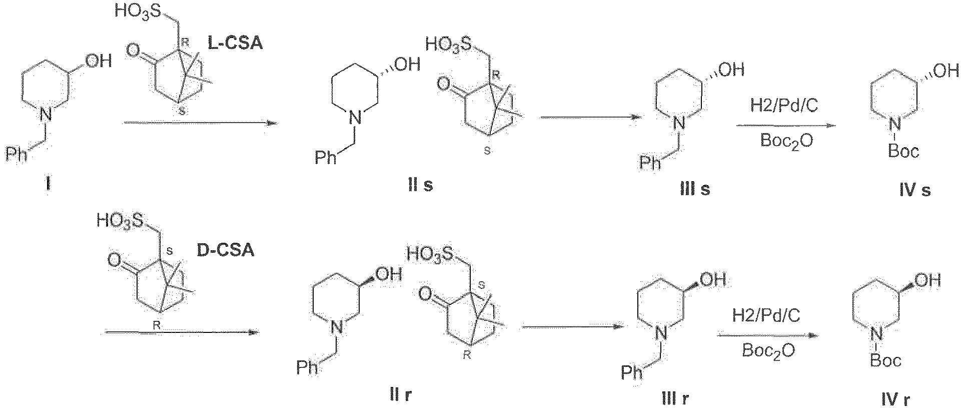

Wherein, a resolution agent of the resolution reaction of step 1) is chiral D-Camphorsulfonic acid (D-CSA) or L-Camphorsulfonic acid (L-CSA), the molar ratio of the chiral D-Camphorsulfonic acid (D-CSA) or L-Camphorsulfonic acid (L-CSA) to the raw material N-benzyl-3-hydroxy piperidine is 0.3∼0.5:1.

Wherein, a solvent system of the resolution reaction of step 1) is: organic alcohols solvent such as methyl alcohol, ethyl alcohol, isopropyl alcohol; organic esters solvent such as ethyl acetate, isopropyl acetate; organic ketones solvent such as 2-butanone, acetone; organic ethers solvent such as THF (Tetrahydrofuran), isopropyl ether, methyl tertiary butyl ether; or any combination thereof.

Wherein, the alkali of step 2) is sodium hydroxide, lithium hydroxide, potassium hydroxide, sodium carbonate, sodium bicarbonate, potassium carbonate or potassium bicarbonate, wherein the molar ratio of the alkali to the raw material N-benzyl-3-hydroxy piperidine is 1∼10:1, preferably is 1∼1.5:1.

Wherein, a solvent system of the reaction of step 3) is: organic alcohols solvent such as methyl alcohol, ethyl alcohol, isopropyl alcohol; organic esters solvent such as ethyl acetate, isopropyl acetate; organic ethers solvent such as THF (Tetrahydrofuran), isopropyl ether, methyl tertiary butyl ether; or any combination thereof.

Wherein, the weight ratio of the palladium-charcoal to substrate (S)-1-benzyl-3-hydroxy piperidine of formula III s or (R)-1-benzyl-3-hydroxy piperidine of formula III r of step 3) is 0.01∼0.5:1; the reaction temperature is between 0°C∼80°C; the reaction pressure is 1.0133 x 10⁵ Pa - 3.04 x 10⁶ Pa (1 atm∼30 atm); wherein the molar ratio of t-butoxycarbonyl acid anhydride to substrate (S)-1-benzyl-3-hydroxy piperidine of formula III s or (R)-1-benzyl-3-hydroxy piperidine of formula III r is 1∼2:1.

The present disclosure also provides a chiral inversion method of the chiral-1-t-butoxycarbonyl-3-hydroxy piperidine, which mainly comprising steps of:
4) Using (R)-1-substituted-3-hydroxy piperidine of formula V r or (S)-1-substituted-3-hydroxy piperidine of formula V s (V) as raw material, and acylating under the action of a substituted sulfonyl chloride to obtain (R)-1-substituted-3-hydroxy piperidine sulfonic ester of formula VI r or (S)-1-substituted-3-hydroxy piperidine sulfonic ester of formula VI s;
5) Substituting the (R)-1-substituted-3-hydroxy piperidine sulfonic ester of formula VI r or (S)-1-substituted-3-hydroxy piperidine sulfonic ester of formula VI s of step 4) with a substituted carboxylate, and structure inversing to obtain (S)-1-substituted-3-hydroxy piperidine carboxylic ester of formula VII s or (R)-1-substituted-3-hydroxy piperidine carboxylic ester of formula VII r;
6) Hydrolyzing the (S)-1-substituted-3-hydroxy piperidine carboxylic ester of formula VII s or (R)-1-substituted-3-hydroxy piperidine carboxylic ester of formula VII r by an alkali to obtain (S)-1-substituted-3-hydroxy piperidine of formula VIII s or (R)-1-substituted-3-hydroxy piperidine of formula VIII r; 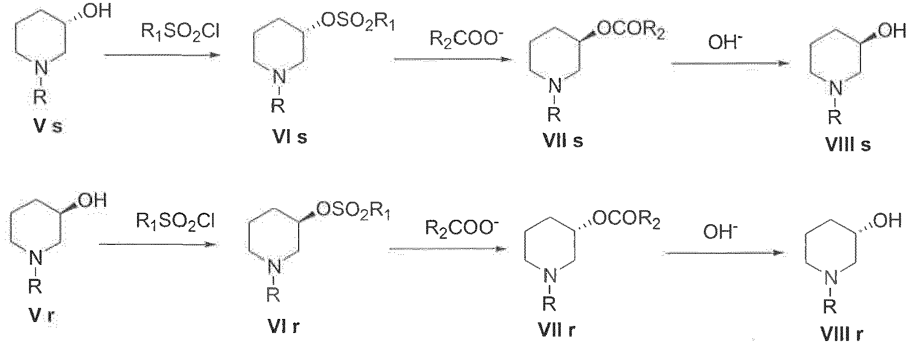
   Wherein, R is benzyl, carboxybenzyl or t-butoxycarbonyl;
   R₁, R₂ are lower paraffin hydrocarbons of C1∼C3, substituted benzyl;
   Wherein, the chiral-1-substituted-3-hydroxy piperidine is the (S)-1-benzyl-3-hydroxy piperidine of formula III s or (R)-1-benzyl-3-hydroxy piperidine of formula III r of step 2) in claim 1; or the (S)-1-t-butoxycarbonyl-3-hydroxy piperidine of formula IV s or (R)-1-t-butoxycarbonyl-3-hydroxy piperidine of formula IV r of step 3) in claim 1.

Wherein, the sulfonyl chloride of step 4) is methanesulfonyl chloride, p-toluenesulfonyl chloride, p-nitrobenzenesulfonyl chloride or p-bromobenzenesulfonyl chloride; a solvent system of the acylation reaction is: organic esters solvent such as ethyl acetate, isopropyl acetate; organic chlorinated solvent such as dichloromethane, trichloromethane, dichloroethane, chlorobenzene; organic ethers solvent such as isopropyl ether, methyl tertiary butyl ether, tetrahydrofuran.

Wherein, the carboxylate of step 5) is sodium acetate or sodium benzoate; a solvent system of the substitution inversion reaction is: organic amides solvent such as DMF (dimethylformamide), formamide; organic esters solvent such as ethyl acetate, isopropyl acetate; organic chlorinated solvent such as dichloromethane, trichloromethane, dichloroethane, chlorobenzene; organic ethers solvent such as isopropyl ether, methyl tertiary butyl ether, tetrahydrofuran; organic alcohols solvent such as methyl alcohol, ethyl alcohol: or a mixture solvent including one or a few types of aforementioned solvents and a mixture solvent system with water.

Wherein, the alkali of the hydrolysis reaction of step 6) is sodium hydroxide, lithium hydroxide, potassium hydroxide; sodium carbonate, potassium carbonate or ammonia water; wherein the molar ratio of the alkali to the raw material N-benzyl-3-hydroxy piperidine of step 4) is 1∼100:1. The solvent of the hydrolysis reaction is water; organic alcohols solvent such as methyl alcohol, ethyl alcohol; organic ethers solvent such as tetrahydrofuran; or including a mixture solvent system thereof.

The method of the present invention solves the problems that it is not easy to obtain 3-hydroxy piperidine in purified form and that it is hard to be industrialized due to its water solubility in the chemical resolution method. Meanwhile, the method of the present invention solves the utilization of the chiral impurities in the resolution process. The synthetic route has not been reported in literature, its reaction is clean, and it is suitable for industrial mass production.

In order to make the abovementioned and other objects, features and advantages of the present invention more clear and easy to understand, preferred embodiments are specially listed in the following specification, detailed explanations are given as follows.

### Specific Mode for Carrying out the Invention

Followings are specific embodiments of the present invention, which further describe the technical solution of the present invention. But the present invention is not limited by these embodiments.

### Step 1) Synthesis of (S) or (R)-1-benzyl-3-hydroxy piperidine camphorsulfonate

### Embodiment 1: Synthesis of (S)-1-benzyl-3-hydroxy piperidine camphorsulfonate

### (1) Synthesis of (S)-1-benzyl-3-hydroxy piperidine camphorsulfonate (one)

N-benzyl-3-hydroxy piperidine (95.6 g, 0.5 mol), 116 ml of isopropyl alcohol solution of 478 ml of isopropyl alcohol added dropwise by L-CSA (58 g, 0.25 mol) were added in a 1000 ml boiling flask-3-neck, stirred for 1 hour at room temperature, precipitated solids appeared, preserved heat for 2 hours at 0°C, filtered, washed by 30 ml of cold isopropyl alcohol, dried to obtain 75 g of (S)-1-benzyl-3-hydroxy piperidine camphorsulfonate. (ee: 95%) (theory: 105.9 g). 95% ee value of (S)-1-benzyl-3-hydroxy piperidine camphorsulfonate was recrystallized by 3 times amount of isopropyl alcohol, and (S)-1-benzyl-3-hydroxy piperidine camphorsulfonate (99% ee) could be obtained.

### (2) Synthesis of (S)-1-benzyl-3-hydroxy piperidine camphorsulfonate (two)

N-benzyl-3-hydroxy piperidine (95.6 g, 0.5 mol), 290 ml of ethyl acetate solution of 478 ml of ethyl acetate added dropwise by L-CSA (58 g, 0.25 mol) were added in a 1000 ml boiling flask-3-neck, stirred for 1 hour at room temperature, precipitated solids appeared, preserved heat for 2 hours at 0°C, filtered, washed by 30 ml of ethyl acetate, dried to obtain 95 g of (S)-1-benzyl-3-hydroxy piperidine camphorsulfonate. (ee: 88%) (theory: 105.9 g).

### (3) Synthesis of (S)-1-benzyl-3-hydroxy piperidine camphorsulfonate (three)

N-benzyl-3-hydroxy piperidine (95.6 g, 0.5 mol), 290 ml of 2-butanone solution of 478 ml of 2-butanone added dropwise by L-CSA (58 g, 0.25 mol) were added in a 1000 ml boiling flask-3-neck, stirred for 1 hour at room temperature, precipitated solids appeared, preserved heat for 2 hours at 0°C, filtered, washed by 30 ml of 2-butanone, dried to obtain 88 g of (S)-1-benzyl-3-hydroxy piperidine camphorsulfonate. (ee: 93%) (theory: 105.9 g).

### (4) Synthesis of (S)-1-benzyl-3-hydroxy piperidine camphorsulfonate (four)

N-benzyl-3-hydroxy piperidine (95.6 g, 0.5 mol), 290 ml of 2-butanone solution of 478 ml of 2-butanone added dropwise by L-CSA (69.6 g, 0.3 mol) were added in a 1000 ml boiling flask-3-neck, stirred for 1 hour at room temperature, precipitated solids appeared, preserved heat for 2 hours at 0°C, filtered, washed by 30 ml of 2-butanone, dried to obtain 108 g of (S)-1-benzyl-3-hydroxy piperidine camphorsulfonate. (ee: 75%) (theory: 105.9 g).

### (5) Synthesis of (S)-1-benzyl-3-hydroxy piperidine camphorsulfonate (five)

N-benzyl-3-hydroxy piperidine (95.6 g, 0.5 mol), 290 ml of 2-butanone solution of 478 ml of 2-butanone added dropwise by L-CSA (52.2 g, 0.225 mol) were added in a 1000 ml boiling flask-3-neck, stirred for 1 hour at room temperature, precipitated solids appeared, preserved heat for 2 hours at 0°C, filtered, washed by 30 ml of 2-butanone, dried to obtain 83 g of (S)-1-benzyl-3-hydroxy piperidine camphorsulfonate. (ee: 95%) (theory: 95.3 g).

### (6) Synthesis of (S)-1-benzyl-3-hydroxy piperidine camphorsulfonate (six)

N-benzyl-3-hydroxy piperidine (95.6 g, 0.5 mol), 478 ml of THF, 290 ml of THF solution added dropwise by L-CSA (58 g, 0.25 mol) were added in a 1000 ml boiling flask-3-neck, stirred for 1 hour at room temperature, precipitated solids appeared, preserved heat for 2 hours at 0°C, filtered, washed by 30 ml of THF, dried to obtain 83 g of (S)-1-benzyl-3-hydroxy piperidine camphorsulfonate. (ee: 92%) (theory: 105.9 g).

### Embodiment 2: Synthesis of (R)-1-benzyl-3-hydroxy piperidine camphorsulfonate

N-benzyl-3-hydroxy piperidine (95.6 g, 0.5 mol), 116 ml of isopropyl alcohol solution of 478 ml of isopropyl alcohol added dropwise by D-CSA (58 g, 0.25 mol) were added in a 1000 ml boiling flask-3-neck, stirred for 1 hour at room temperature, precipitated solids appeared, preserved heat for 2 hours at 0°C, filtered, washed by 30 ml of cold isopropyl alcohol, dried to obtain 75 g of (R)-1-benzyl-3-hydroxy piperidine camphorsulfonate. (ee: 95%) (theory: 105.9 g). 95% ee value of (R)-1-benzyl-3-hydroxy piperidine camphorsulfonate was recrystallized by 3 times amount of isopropyl alcohol, and (R)-1-benzyl-3-hydroxy piperidine camphorsulfonate (99% ee) could be obtained.

### Step 2) Synthesis of (S) or (R)-1-benzyl-3-hydroxy piperidine

### Embodiment 3: Synthesis of (S)-1-benzyl-3-hydroxy piperidine

### (1) Synthesis of (S)-1-benzyl-3-hydroxy piperidine (one)

(S)-1-benzyl-3-hydroxy piperidine camphorsulfonate (99% ee, 84.7 g, 0.2 mol) prepared in any one of (1), (2), (3) of the embodiment 1, 423 ml of dichloromethane were added in a 1000 ml boiling flask-3-neck, added dropwise by IN sodium hydroxide aqueous solution (210 ml), stirred for 1 hour at room temperature, removed aqueous phase, dried by organic phase anhydrous sodium sulfate, concentrated to obtain 38 g of (S)-1-benzyl-3-hydroxy piperidine. (ee: 99%; LC-MS: m/e=191.3) (theory: 38.25 g).

### (2) Synthesis of (S)-1-benzyl-3-hydroxy piperidine (two)

(S)-1-benzyl-3-hydroxy piperidine camphorsulfonate (99% ee, 84.7 g, 0.2 mol) prepared in any one of (4), (5), (6) of the embodiment 1, 423 ml of ethyl acetate were added in a 1000 ml boiling flask-3-neck, added dropwise by IN potassium carbonate aqueous solution (150 ml), stirred for 1 hour at room temperature, removed aqueous phase, dried by organic phase anhydrous sodium sulfate, concentrated to obtain 36 g of (S)-1-benzyl-3-hydroxy piperidine. (ee: 99%; LC-MS: m/e=191.3) (theory: 38.25 g).

### Embodiment 4: Synthesis of (R)-1-benzyl-3-hydroxy piperidine

(R)-1-benzyl-3-hydroxy piperidine camphorsulfonate (99%ee, 84.7 g, 0.2 mol) prepared in the embodiment 2, 423 ml of dichloromethane were added in a 1000 ml boiling flask-3-neck, added dropwise by IN sodium hydroxide aqueous solution (210 ml), stirred for 1 hour at room temperature, removed aqueous phase, dried by organic phase anhydrous sodium sulfate, concentrated to obtain 38 g of (R)-1-benzyl-3-hydroxy piperidine. (ee: 99%; LC-MS: m/e=191.3) (theory: 38.25 g).

### Step 3): Synthesis of (S) or (R)-1-t-butoxycarbonyl-3-hydroxy piperidine

### Embodiment 5: Synthesis of (S)-1-t-butoxycarbonyl-3-hydroxy piperidine

### (1) Synthesis of (S)-1-t-butoxycarbonyl-3-hydroxy piperidine (one)

(S)-1-benzyl-3-hydroxy piperidine (38.25 g, 0.2 mol) prepared in the embodiment 3, 200 ml of methyl alcohol, 10% palladium-charcoal (3.8 g) and t-butoxycarbonyl acid anhydride (43.6 g, 0.2 mol) were added in a 1000 ml high pressure reactor, under hydrogen atmosphere, the pressure was controlled to 5.066 x 10⁵ Pa (∼5 atm), ∼40°C, reacted for 15 hours, HPLC raw material ((S)-1-benzyl-3-hydroxy piperidine)<0.2%; reaction liquid was cooled to room temperature, stopped reaction, filtered, palladium-charcoal was recycled, filtrate was concentrated to dry under reduced pressure, and added in ethyl acetate/n-hexane to recrystallize, solid was separated out, filtered; the solid was washed by a little n-hexane; dried to obtain ∼36 g of product (S)-1-t-butoxycarbonyl-3-hydroxy piperidine. (theory: 40.2 g) (HPLC content ∼99%; ee: 99%; LC-MS: m/e=201.3).

### (2) Synthesis of (S)-1-t-butoxycarbonyl-3-hydroxy piperidine (two)

(S)-1-benzyl-3-hydroxy piperidine (38.25 g, 0.2 mol) prepared in the embodiment 3, 200 ml of ethyl acetate, 10% palladium-charcoal (1.9 g) and t-butoxycarbonyl acid anhydride (48 g, 0.22 mol) were added in a 1000 ml high pressure reactor, under hydrogen atmosphere, the pressure was controlled to 1.013 x 10⁶ Pa (∼10 atm), ∼50°C, reacted for 15 hours, HPLC raw material ((S)-1-benzyl-3-hydroxy piperidine)<0.2%; reaction liquid was cooled to room temperature, stopped reaction, filtered, palladium-charcoal was recycled, filtrate was concentrated to 1/2 under reduced pressure, and added in n-hexane to recrystallize, solid was separated out, filtered; the solid was washed by a little n-hexane; dried to obtain ∼37 g of product (S)-1-t-butoxycarbonyl-3-hydroxy piperidine, (theory: 40.2 g) (HPLC content ∼99%; ee: 99%; LC-MS: m/e=201.3).

### (3) Synthesis of (S)-1-t-butoxycarbonyl-3-hydroxy piperidine (three)

(S)-1-benzyl-3-hydroxy piperidine (38.25 g, 0.2 mol) prepared in (2) of the embodiment 3, 200 ml of THF, 10% palladium-charcoal (0.95 g) and t-butoxycarbonyl acid anhydride (54.5 g, 0.25 mol) were added in a 1000 ml high pressure reactor, under hydrogen atmosphere, the pressure was controlled to 2.026 x 10⁶ Pa (∼20 atm), ∼40°C, reacted for 15 hours, HPLC raw material ((S)-1-benzyl-3-hydroxy piperidine)<0.2%; reaction liquid was cooled to room temperature, stopped reaction, filtered, palladium-charcoal was recycled, filtrate was concentrated to dry under reduced pressure, and added in ethyl acetate/n-hexane to recrystallize, solid was separated out, filtered; the solid was washed by a little n-hexane; dried to obtain ∼38 g of product (S)-1-t-butoxycarbonyl-3-hydroxy piperidine, (theory: 40.2 g) (HPLC content ∼99%; ee: 99%; LC-MS: m/e=201.3).

### Embodiment 6: Synthesis of (R)-1-t-butoxycarbonyl-3-hydroxy piperidine

(R)-1-benzyl-3-hydroxy piperidine (38.25 g, 0.2 mol) prepared in the embodiment 4, 200 ml of methyl alcohol, 10% palladium-charcoal (0.95 g) and t-butoxycarbonyl acid anhydride (43.6 g, 0.2 mol) were added in a 1000 ml high pressure reactor, under hydrogen atmosphere, the pressure was controlled to 5.066 x 10⁵ Pa (∼5 atm), ∼60°C, reacted for 15 hours, HPLC raw material ((R)-1-benzyl-3-hydroxy piperidine)<0.2%; reaction liquid was cooled to room temperature, stopped reaction, filtered, palladium-charcoal was recycled, filtrate was concentrated to dry under reduced pressure, and added in ethyl acetate/n-hexane to recrystallize, solid was separated out, filtered; the solid was washed by a little n-hexane; dried to obtain ∼36 g of product (R)-1-t-butoxycarbonyl-3-hydroxy piperidine. (theory: 40.2 g) (HPLC content ∼99%; ee: 99%; LC-MS: m/e=201.3).

### Step 4) Synthesis of (R) or (S)-1-substituted-3-hydroxy piperidine sulfonic ester

### Embodiment 7:

### (1) Synthesis of (R)-1-t-butoxycarbonyl-3-methanesulfonic acid piperidine (one)

(R)-1-t-butoxycarbonyl-3-hydroxy piperidine (30.2 g, 0.15 mol) prepared in the embodiment 6, 180 ml of dichloromethane, triethylamine (18.2 g; 0.18 mol) were added in a 500 ml boiling flask-3-neck, controlled to 0∼10°C, added dropwise by methanesulfonyl chloride (18.9 g, 0.165 mol), stirred for 1 hour at room temperature, added water to quench, removed aqueous phase, organic phase was water-washed, and dried by anhydrous sodium sulfate, concentrated to obtain 39.8 g of (R)-1-t-butoxycarbonyl-3-methanesulfonic acid piperidine. (yield: 95%; LC-MS: m/e=279.1) (theory: 41.9 g).

### (2) Synthesis of (R)-1-t-butoxycarbonyl-3-methanesulfonic acid piperidine (two)

(R)-1-t-butoxycarbonyl-3-hydroxy piperidine (30.2 g, 0.15 mol) prepared in the embodiment 6, 180 ml of isopropyl acetate, triethylamine (18.2 g; 0.18 mol) were added in a 500 ml boiling flask-3-neck, controlled to 0∼10°C, added dropwise by methanesulfonyl chloride (18.9 g, 0.165 mol), stirred for 1 hour at room temperature, added water to quench, removed aqueous phase, organic phase was water-washed, and dried by anhydrous sodium sulfate, concentrated to obtain 39.8 g of (R)-1-t-butoxycarbonyl-3-methanesulfonic acid piperidine. (yield: 95%; LC-MS: m/e=279.1) (theory: 41.9 g).

### Embodiment 8: Synthesis of (R)-1-benzyl-3-methanesulfonic acid piperidine

(R)-1-benzyl-3-hydroxy piperidine (28.7 g, 0.15 mol) prepared in the embodiment 4, 180 ml of dichloromethane, triethylamine (18.2 g; 0.18 mol) were added in a 500 ml boiling flask-3-neck, controlled to 0∼10°C, added dropwise by methanesulfonyl chloride (18.9 g, 0.165 mol), stirred for 1 hour at room temperature, added water to quench, removed aqueous phase, organic phase was water-washed, and dried by anhydrous sodium sulfate, concentrated to obtain 37.2 g of (R)-1-benzyl-3-methanesulfonic acid piperidine. (yield: 92%; LC-MS: m/e=269.4) (theory: 40.4 g).

### Embodiment 9: Synthesis of (R)-1-t-butoxycarbonyl-3-p-nitrobenzenesulphonic acid piperidine

(R)-1-t-butoxycarbonyl-3-hydroxy piperidine (30.2 g, 0.15 mol) prepared in the embodiment 6, 180 ml of dichloromethane, triethylamine (18.2 g; 0.18 mol) were added in a 500 ml boiling flask-3-neck, controlled to 0∼10°C, added dropwise by dichloromethane solution of p-nitrobenzenesulfonyl chloride (36.6 g, 0.165 mol), stirred overnight at room temperature, added water to quench, removed aqueous phase, organic phase was water-washed, and dried by anhydrous sodium sulfate, concentrated to obtain 54.5 g of (R)-1-t-butoxycarbonyl-3-p-nitrobenzenesulphonic acid piperidine. (yield: 94%; LC-MS: m/e=386.4) (theory: 58.0 g).

### Embodiment 10: Synthesis of (R)-1-t-butoxycarbonyl-3-p-toluenesulfonic acid piperidine

(R)-1-t-butoxycarbonyl-3-hydroxy piperidine (30.2 g, 0.15 mol) prepared in the embodiment 6, 180 ml of dichloroethane, triethylamine (18.2 g; 0.18 mol) were added in a 500 ml boiling flask-3-neck, added dropwise by dichloroethane solution of p-toluenesulfonyl chloride (31.4 g, 0.165 mol) at room temperature, reacted overnight at 50∼60°C, added water to quench, removed aqueous phase, organic phase was water-washed, and dried by anhydrous sodium sulfate, concentrated to obtain 48.0 g of (R)-1-t-butoxycarbonyl-3-p-toluenesulfonic acid piperidine. (yield: 90%; LC-MS: m/e=355.5) (theory: 53.3 g).

### Embodiment 11: Synthesis of (S)-1-t-butoxycarbonyl-3-methanesulfonic acid piperidine

(S)-1-t-butoxycarbonyl-3-hydroxy piperidine (30.2 g, 0.15 mol) prepared in any one of the embodiment 5(1)(2)(3), 180 ml of dichloromethane, triethylamine (18.2 g; 0.18 mol) were added in a 500 ml boiling flask-3-neck, controlled to 0∼10°C, added dropwise by methanesulfonyl chloride (18.9 g, 0.165 mol), stirred for 1 hour at room temperature, added water to quench, removed aqueous phase, organic phase was water-washed, and dried by anhydrous sodium sulfate, concentrated to obtain 39.8 g of (S)-1-t-butoxycarbonyl-3-methanesulfonic acid piperidine. (yield: 95%; LC-MS: m/e=279.1) (theory: 41.9 g).

### Step 5) Inversing to obtain (S) or (R)-1-substituted-3-hydroxy piperidine carboxylic ester

### Embodiment 12: Synthesis of (S)-1-t-butoxycarbonyl-3-acetoxy piperidine

(R)-1-t-butoxycarbonyl-3-methanesulfonic acid piperidine (27.9 g, 0.1 mol) prepared in (1) or (2) of the embodiment 7, 100 ml of DMF, sodium acetate (16.4 g; 0.2 mol) were added in a 500 ml boiling flask-3-neck, reacted overnight at 60°C, added water to quench, extracted by ethyl acetate, organic phase was water-washed, concentrated to obtain crude product of (S)-1-t-butoxycarbonyl-3-acetoxy piperidine. (yield: ∼100%; LC-MS: m/e=243.3) (theory: 24.3 g).

### Embodiment 13:

### (1) Synthesis of (S)-1-t-butoxycarbonyl-3-benzoyloxy piperidine (one)

(R)-1-t-butoxycarbonyl-3-methanesulfonic acid piperidine (27.9 g, 0.1 mol) prepared in (1) or (2) of the embodiment 7, 100 ml of DMF, sodium benzoate (28.8 g; 0.2 mol) were added in a 500 ml boiling flask-3-neck, reacted overnight at 80°C, reaction liquid was cooled, added water to quench, extracted by dichloromethane, organic phase was water-washed, concentrated to obtain crude product of (S)-1-t-butoxycarbonyl-3-benzoyloxy piperidine. (yield: ∼100%; LC-MS: m/e=305.4) (theory: 30.5 g).

### (2) Synthesis of (S)-1-t-butoxycarbonyl-3-benzoyloxy piperidine (two)

(R)-1-t-butoxycarbonyl-3-p-nitrobenzenesulphonic acid piperidine (38.6 g, 0.1 mol) prepared in the embodiment 9, 100 ml of DMF, sodium benzoate (28.8 g; 0.2 mol) were added in a 500 ml boiling flask-3-neck, reacted overnight at 80°C, reaction liquid was cooled, added water to quench, extracted by dichloromethane, organic phase was water-washed, concentrated to obtain crude product of (S)-1-t-butoxycarbonyl-3-benzoyloxy piperidine. (yield: ∼100%; LC-MS: m/e=305.4) (theory: 30.5 g).

### (3) Synthesis of (S)-1-t-butoxycarbonyl-3-benzoyloxy piperidine (three)

(R)-1-t-butoxycarbonyl-3-p-toluenesulfonic acid piperidine (35.5 g, 0.1 mol) prepared in the embodiment 10, 100 ml of DMF, sodium benzoate (28.8 g; 0.2 mol) were added in a 500 ml boiling flask-3-neck, reacted overnight at 90°C, reaction liquid was cooled, added water to quench, extracted by dichloromethane, organic phase was water-washed, concentrated to obtain crude product of (S)-1-t-butoxycarbonyl-3-benzoyloxy piperidine. (yield: ∼100%; LC-MS: m/e=305.4) (theory: 30.5 g).

### Embodiment 14: Synthesis of (S)-1-benzyl-3-benzoyloxy piperidine

(R)-1-benzyl-3-methanesulfonic acid piperidine (26.9 g, 0.1 mol) prepared in the embodiment 8, 100 ml of DMF, sodium benzoate (28.8 g; 0.2 mol) were added in a 500 ml boiling flask-3-neck, reacted overnight at 90°C, reaction liquid was cooled, added water to quench, extracted by dichloromethane, organic phase was water-washed, concentrated to obtain crude product of (S)-1-benzyl-3-benzoyloxy piperidine. (yield: ∼100%; LC-MS: m/e=295.4) (theory: 29.5 g).

### Embodiment 15: Synthesis of (R)-1-t-butoxycarbonyl-3-acetoxy piperidine

(S)-1-t-butoxycarbonyl-3-methanesulfonic acid piperidine (26.9 g, 0.1 mol) prepared in the embodiment 11, 100 ml of DMF, sodium acetate (16.4 g; 0.2 mol) were added in a 500 ml boiling flask-3-neck, reacted overnight at 90°C, reaction liquid was cooled, added water to quench, extracted by dichloromethane, organic phase was water-washed, concentrated to obtain crude product of (R)-1-t-butoxycarbonyl-3-acetoxy piperidine. (yield: ∼100%; LC-MS: m/e=233.3) (theory: 23.3 g).

### Step 6): Hydrolyzing by alkali to obtain (S) or (R)-1-substituted-3-hydroxy piperidine

### Embodiment 16: Synthesis of (S)-1-benzyl-3-hydroxy piperidine

The crude product of (S)-1-benzyl-3-benzoyloxy piperidine (0.1 mol) prepared in the embodiment 14, 50 ml of methyl alcohol; 50 ml of water and 6 g of sodium hydroxide (0.15 mol) were added in a 500 ml boiling flask-3-neck, reacted overnight at room temperature, reaction liquid was diluted by adding water, extracted by dichloromethane, organic phase was water-washed, concentrated, ∼18 g of product (S)-1-benzyl-3-hydroxy piperidine was obtained. (HPLC content ∼99%; ee: 99%; LC-MS: m/e=191.3) (theory: 19.1 g).

### Embodiment 17: Synthesis of (S)-1-t-butoxycarbonyl-3-hydroxy piperidine

### (1) Synthesis of (S)-1-t-butoxycarbonyl-3-hydroxy piperidine (four)

The crude product of (S)-1-t-butoxycarbonyl-3-acetoxy piperidine (0.1 mol) prepared in the embodiment 12, 50 ml of methyl alcohol; 50 ml of water and 6 g of sodium hydroxide (0.15 mol) were added in a 500 ml boiling flask-3-neck, reacted overnight at room temperature, reaction liquid was diluted by adding water, extracted by dichloromethane, organic phase was water-washed, concentrated, recrystallized by ethyl acetate/n-hexane, dried to obtain ∼16 g of product (R)-1-t-butoxycarbonyl-3-hydroxy piperidine. (HPLC content ∼99%; ee: 99%; LC-MS: m/e=201.3) (theory: 20.1 g).

### (2) Synthesis of (S)-1-t-butoxycarbonyl-3-hydroxy piperidine (five)

The crude product of (S)-1-t-butoxycarbonyl-3-acetoxy piperidine (0.1 mol) prepared in the embodiment 12, 50 ml of methyl alcohol; 50 ml of water and 6 g of sodium hydroxide (0.15 mol) were added in a 500 ml boiling flask-3-neck, reacted overnight at room temperature, reaction liquid was diluted by adding water, extracted by dichloromethane, organic phase was water-washed, concentrated, recrystallized by ethyl acetate/n-hexane, dried to obtain -16 g of product (R)-1-t-butoxycarbonyl-3-hydroxy piperidine. (HPLC content ∼99%; ee: 99%; LC-MS: m/e=201.3) (theory: 20.1 g).

### (3) Synthesis of (S)-1-t-butoxycarbonyl-3-hydroxy piperidine (six)

The crude product of (S)-1-t-butoxycarbonyl-3-acetoxy piperidine (0.1 mol) prepared in the embodiment 12, 50 ml of methyl alcohol; 50 ml of water and 9.1 g of potassium hydroxide (∼0.15 mol) were added in a 500 ml boiling flask-3-neck, reacted overnight at room temperature, reaction liquid was diluted by adding water, extracted by dichloromethane, organic phase was water-washed, concentrated, recrystallized by ethyl acetate/n-hexane, dried to obtain ∼15 g of product (R)-1-t-butoxycarbonyl-3-hydroxy piperidine. (HPLC content ∼99%; ee: 99%; LC-MS: m/e=201.3) (theory: 20.1 g).

### (4) Synthesis of (S)-1-t-butoxycarbonyl-3-hydroxy piperidine (seven)

The crude product of (S)-1-t-butoxycarbonyl-3-acetoxy piperidine (0.1 mol) prepared in the embodiment 12, 50 ml of THF; 50 ml of water and 6 g of sodium hydroxide (0.15 mol) were added in a 500 ml boiling flask-3-neck, reacted overnight at room temperature, reaction liquid was diluted by adding water, extracted by dichloromethane, organic phase was water-washed, concentrated, recrystallized by ethyl acetate/n-hexane, dried to obtain ∼16 g of product (R)-1-t-butoxycarbonyl-3-hydroxy piperidine. (HPLC content ∼99%; ee: 99%; LC-MS: m/e=201.3) (theory: 20.1 g).

### (5) Synthesis of (S)-1-t-butoxycarbonyl-3-hydroxy piperidine (eight)

The crude product of (S)-1-t-butoxycarbonyl-3-acetoxy piperidine (0.1 mol) prepared in the embodiment 12, 100 ml of ethyl alcohol; 10 ml of stronger ammonia water were added in a 500 ml boiling flask-3-neck, reacted overnight at room temperature, reaction liquid was concentrated under reduced pressure, diluted by adding water, extracted by dichloromethane, organic phase was water-washed, concentrated, recrystallized by ethyl acetate/n-hexane, dried to obtain ∼16 g of product (R)-1-t-butoxycarbonyl-3-hydroxy piperidine. (HPLC content ∼99%; ee: 99%; LC-MS: m/e=201.3) (theory: 20.1 g).

### Embodiment 18: Synthesis of (R)-1-t-butoxycarbonyl-3-hydroxy piperidine

The crude product of (R)-1-t-butoxycarbonyl-3-acetoxy piperidine (0.1 mol) prepared in the embodiment 15, 50 ml of methyl alcohol; 50 ml of water and 6 g of sodium hydroxide (0.15 mol) were added in a 500 ml boiling flask-3-neck, reacted overnight at room temperature, reaction liquid was diluted by adding water, extracted by dichloromethane, organic phase was water-washed, concentrated, recrystallized by ethyl acetate/n-hexane, dried to obtain ∼16 g of product (R)-1-t-butoxycarbonyl-3-hydroxy piperidine. (HPLC content ∼99%; ee: 99%; LC-MS: m/e=201.3) (theory: 20.1 g).

Although the present invention has been disclosed as above by preferred embodiments, they shall not be used to limit the present invention. Slight modifications and improvements can be made by any person skilled in the art.

## Claims

1. A method for the preparation of chiral 1-t-butoxycarbonyl-3-hydroxy piperidine, wherein the method comprises the steps of:
1) Using N-benzyl-3-hydroxy piperidine of formula I as raw material, and resolving it under the action of chiral camphorsulfonic acid (CSA) to obtain (S)-1-benzyl-3-hydroxy piperidine camphorsulfonate of formula II s or (R)-1-benzyl-3-hydroxy piperidine camphorsulfonate of formula II r;
2) Dissociating the (S)-1-benzyl-3-hydroxy piperidine camphorsulfonate of formula II s or (R)-1-benzyl-3-hydroxy piperidine camphorsulfonate of formula II r of step 1) by an alkali to obtain (S)-1-benzyl-3-hydroxy piperidine of formula III s or (R)-1-benzyl-3-hydroxy piperidine of formula III r;
3) Catalytic hydrogenating and debenzylating the (S)-1-benzyl-3-hydroxy piperidine of formula III s or (R)-1-benzyl-3-hydroxy piperidine of formula III r of step 2) by palladium-charcoal, meanwhile, protecting by t-butoxycarbonyl acid anhydride to obtain (S)-1-t-butoxycarbonyl-3-hydroxy piperidine of formula IV s or (R)-1-butoxycarbonyl-3-hydroxy piperidine of formula IV r.

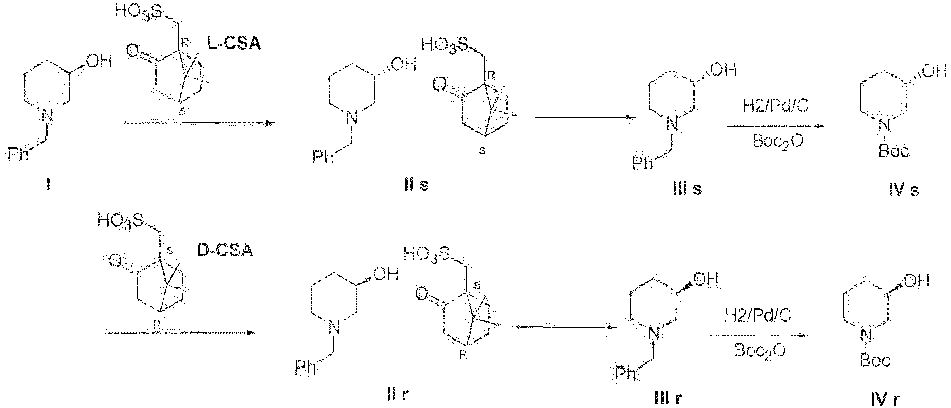

2. The method of claim 1, wherein the chiral Camphorsulfonic acid of step 1 is D-Camphorsulfonic acid (D-CSA) or L-Camphorsulfonic acid (L-CSA), wherein the molar ratio of the chiral D-Camphorsulfonic acid (D-CSA) or L-Camphorsulfonic acid (L-CSA) to the raw material N-benzyl-3-hydroxy piperidine is 0.3-0.5:1.

3. The method of claim 1, wherein a solvent system of the resolution reaction of step 1) is: organic alcohol solvent; organic ester solvent; organic ketone solvent; organic ether solvent; or any combination thereof.

4. The method of claim 3, wherein the organic alcohol solvent is methyl alcohol, ethyl alcohol or isopropyl alcohol.

5. The method of claim 3, wherein the organic ester solvent is ethyl acetate or isopropyl acetate.

6. The method of claim 3, wherein the organic ketone solvent is 2-butanone or acetone.

7. The method of claim 3, wherein the organic ether solvent is isopropyl ether or methyl tertiary butyl ether.

8. The method of claim 1, wherein the alkali of step 2) is sodium hydroxide, lithium hydroxide, potassium hydroxide, sodium carbonate, sodium bicarbonate, potassium carbonate or potassium bicarbonate, wherein the molar ratio of the alkali to the raw material N-benzyl-3-hydroxy piperidine is 1-10:1.

9. The method of claim 1, wherein a solvent system of the reaction of step 3) is: organic alcohol solvent; organic ester solvent; organic ether solvent; or any combination thereof.

10. The method of claim 9, wherein the organic alcohol solvent is methyl alcohol, ethyl alcohol or isopropyl alcohol.

11. The method of claim 9, wherein the organic ester solvent is ethyl acetate or isopropyl acetate.

12. The method of claim 9, wherein the organic ether solvent is isopropyl ether or methyl tertiary butyl ether.

13. The method of claim 1, wherein the weight ratio of the palladium-charcoal to substrate (S)-1-benzyl-3-hydroxy piperidine of formula III s or (R)-1-benzyl-3-hydroxy piperidine of formula III r of step 3) is 0.01-0.5:1; the reaction temperature is between 0°C-80°C; the reaction pressure is 1.0133 x 10⁵ Pa - 3.04 x 10⁶ Pa (1 atm -30 atm); wherein the molar ratio of t-butoxycarbonyl acid anhydride to substrate (S)-1-benzyl-3-hydroxy piperidine of formula III s or (R)-1-benzyl-3-hydroxy piperidine of formula III r is 1-2:1.

## Patentansprüche

1. Verfahren zur Herstellung von chiralem 1-t-Butoxycarbonyl-3-hydroxypiperidin, wobei das Verfahren die folgenden Schritte umfasst:
1) Verwendung von N-Benzyl-3-hydroxypiperidin der Formel I als Ausgangsmaterial und dessen Auflösen unter Einwirkung chiraler Camphersulfonsäure (CSA) zum Erhalt von (S)-1-Benzyl-3-hydroxypiperidin-Camphersulfonat der Formel II s oder (R)-1-Benzyl-3-hydroxypiperidin-Camphersulfonat der Formel II r;
2) Spaltung des (S)-1-Benzyl-3-hydroxypiperidin-Camphersulfonats der Formel II s oder (R)-1-Benzyl-3-hydroxypiperidin-Camphersulfonats der Formel II r von Schritt 1) durch ein Alkali zum Erhalt von (S)-1-Benzyl-3-hydroxypiperidin der Formel III s oder (R)-1-Benzyl-3-hydroxypiperidin der Formel III r;
3) katalytische Hydrierung und Debenzylierung des (S)-1-Benzyl-3-hydroxypiperidins der Formel III s oder (R)-1-Benzyl-3-hydroxypiperidins der Formel III r von Schritt 2) durch Palladiumkohle, zwischenzeitlich geschützt durch t-Butoxycarbonylsäureanhydrid, zum Erhalt von (S)-1-t-Butoxycarbonyl-3-hydroxypiperidin der Formel IV s oder (R)-1-Butoxycarbonyl-3-hydroxypiperidin der Formel IV r.

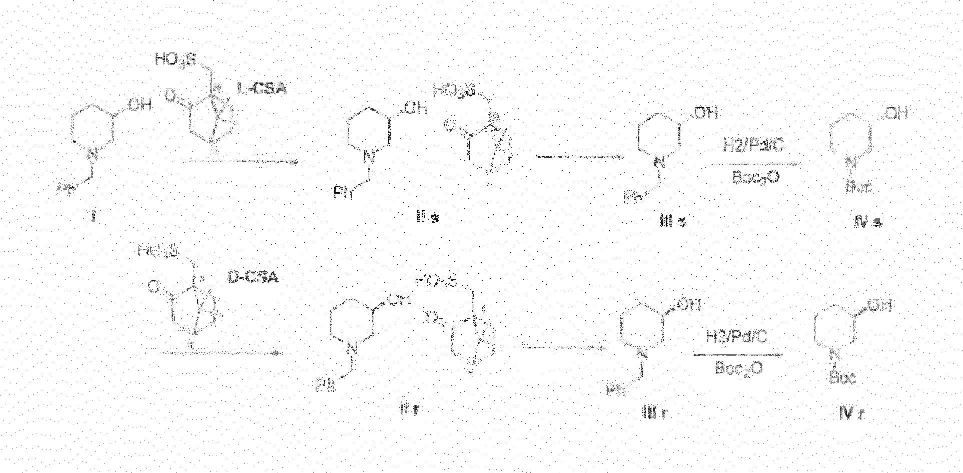

2. Verfahren nach Anspruch 1, wobei die chirale Camphersulfonsäure aus Schritt 1) D-Camphersulfonsäure (D-CSA) oder L-Camphersulfonsäure (L-CSA) ist, wobei das Molverhältnis der chiralen D-Camphersulfonsäure (D-CSA) oder L-Camphersulfonsäure (L-CSA) zum Ausgangsmaterial N-Benzyl-3-hydroxypiperidin 0,3-0,5:1 beträgt.

3. Verfahren nach Anspruch 1, wobei ein Lösungsmittelsystem der Auflösungsreaktion von Schritt 1) ist: ein organisches Alkohollösungsmittel; ein organisches Esterlösungsmittel; ein organisches Ketonlösungsmittel; ein organisches Etherlösungsmittel; oder eine beliebige Kombination davon.

4. Verfahren nach Anspruch 3, wobei das organische Alkohollösungsmittel entweder Methylalkohol, Ethylalkohol oder Isopropylalkohol ist.

5. Verfahren nach Anspruch 3, wobei das organische Esterlösungsmittel entweder Ethylacetat oder Isopropylacetat ist.

6. Verfahren nach Anspruch 3, wobei das organische Ketonlösungsmittel entweder 2-Butanon oder Aceton ist.

7. Verfahren nach Anspruch 3, wobei das organische Etherlösungsmittel entweder Isopropylether oder Methyl-tertiär-Butylether ist.

8. Verfahren nach Anspruch 1, wobei das Alkali von Schritt 2) Natriumhydroxid, Lithiumhydroxid, Kaliumhydroxid, Natriumcarbonat, Natriumbicarbonat, Kaliumcarbonat oder Kaliumbicarbonat ist, wobei das Molverhältnis vom Alkali zum Ausgangsmaterial N-Benzyl-3-hydroxypiperidin 1-10:1 beträgt.

9. Verfahren nach Anspruch 1, wobei ein Lösungsmittelsystem der Reaktion von Schritt 3) ist: ein organisches Alkohollösungsmittel; ein organisches Esterlösungsmittel; ein organisches Etherlösungsmittel; oder eine beliebige Kombination davon.

10. Verfahren nach Anspruch 9, wobei das organische Alkohollösungsmittel entweder Methylalkohol, Ethylalkohol oder Isopropylalkohol ist.

11. Verfahren nach Anspruch 9, wobei das organische Esterlösungsmittel entweder Ethylacetat oder Isopropylacetat ist.

12. Verfahren nach Anspruch 9, wobei das organische Etherlösungsmmittel entweder Isopropylether oder Methyl-tertiär-Butylether ist.

13. Verfahren nach Anspruch 1, wobei das Gewichtsverhältnis der Palladiumkohle zum Substrat (S)-1-Benzyl-3-hydroxypiperidin der Formel III s oder (R)-1-Benzyl-3-hydroxypiperidin der Formel III r von Schritt 3) 0,01-0,5:1 beträgt; die Reaktionstemperatur zwischen 0°C-80°C liegt; der Reaktionsdruck 1,0133 x 10⁵ Pa - 3,04 x 10⁶ Pa (1 atm - 30 atm) beträgt; wobei das Molverhältnis von t-Butoxycarbonylsäureanhydrid zum Substrat (S)-1-Benzyl-3-hydroxypiperidin der Formel III s oder (R)-1-Benzyl-3-hydroxypiperidin der Formel III r 1-2:1 beträgt.

## Revendications

1. Procédé de préparation de 1-t-butoxycarbonyl-3-hydroxy pipéridine chirale, dans lequel le procédé comprend les étapes consistant à :
1) utiliser la N-benzyl-3-hydroxy pipéridine de la formule I comme matière première et la résoudre sous l'action de l'acide camphosulfonique chiral (CSA) pour obtenir le camphosulfonate de (S)-1-benzyl-3-hydroxy pipéridine de la formule II s ou le camphosulfonate de (R)-1-benzyl-3-hydroxy pipéridine de la formule II r;
2) dissocier le camphosulfonate de (S)-1-benzyl-3-hydroxy pipéridine de la formule II s ou le camphosulfonate de (R)-1-benzyl-3-hydroxy pipéridine de la formule II r de l'étape 1) par un alcali pour obtenir la (S)-1-benzyl-3-hydroxy pipéridine de la formule III s ou la (R)-1-benzyl-3-hydroxy-pipéridine de la formule III r;
3) hydrogéner de manière catalytique et débenzyler la (S)-1-benzyl-3-hydroxy pipéridine de la formule III s ou la (R)-1-benzyl-3-hydroxy pipéridine de la formule III r de l'étape 2) par du charbon palladié, entretemps, protéger par de l'anhydride d'acide t-butoxycarbonylique pour obtenir la (S)-1-t-butoxycarbonyl-3-hydroxy pipéridine de la formule IV s ou la (R)-1-butoxycarbonyl-3-hydroxy pipéridine de la formule IV r.

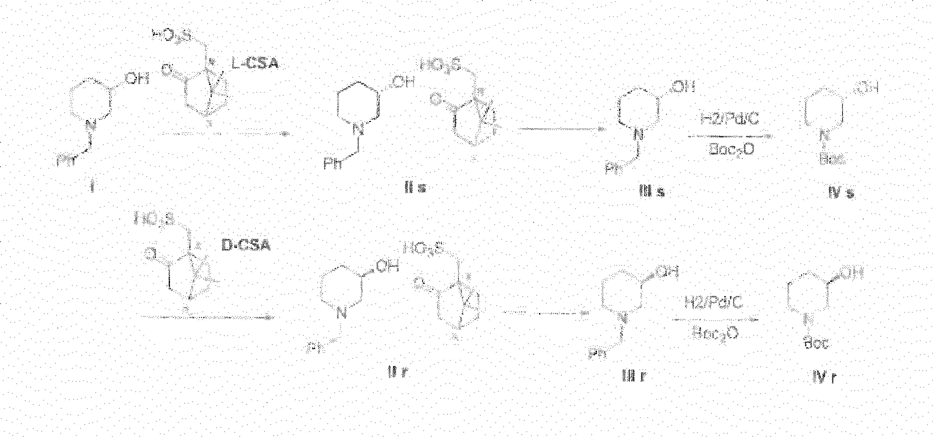

2. Procédé selon la revendication 1, dans lequel l'acide camphosulfonique chiral de l'étape 1 est l'acide D-camphosulfonique (D-CSA) ou l'acide L-camphosulfonique (L-CSA), dans lequel le rapport molaire entre l'acide D-camphosulfonique chiral (D-CSA) ou l'acide L-camphosulfonique (L-CSA) et la matière première N-benzyl-3-hydroxy pipéridine est de 0,3 à 0,5:1.

3. Procédé selon la revendication 1, dans lequel un système de solvant de la réaction de résolution de l'étape 1) est: un solvant à base d'alcool organique; un solvant à base d'ester organique; un solvant à base de cétone organique; un solvant à base d'éther organique; ou toute combinaison de ces derniers.

4. Procédé selon la revendication 3, dans lequel le solvant à base d'alcool organique est l'alcool méthylique, l'alcool éthylique ou l'alcool isopropylique.

5. Procédé selon la revendication 3, dans lequel le solvant à base d'ester organique est l'acétate d'éthyle ou l'acétate d'isopropyle.

6. Procédé selon la revendication 3, dans lequel le solvant à base de cétone organique est le 2-butanone ou l'acétone.

7. Procédé selon la revendication 3, dans lequel le solvant à base d'éther organique est l'éther isopropylique ou le méthyl-tertio-butyl-éther.

8. Procédé selon la revendication 1, dans lequel l'alcali de l'étape 2) est l'hydroxyde de sodium, l'hydroxyde de lithium, l'hydroxyde de potassium, le carbonate de sodium, le bicarbonate de sodium, le carbonate de potassium ou le bicarbonate de potassium, dans lequel le rapport molaire entre l'alcali et la matière première N-benzyl-3-hyrlroxy pipéridine est de 1 à 10:1.

9. Procédé selon la revendication 1, dans lequel un système de solvant de la réaction de l'étape 3) est: un solvant à base d'alcool organique; un solvant à base d'ester organique; un solvant à base d'éther organique; ou toute combinaison de ces derniers.

10. Procédé selon la revendication 9, dans lequel le solvant à base d'alcool organique est l'alcool méthylique, l'alcool éthylique ou l'alcool isopropylique.

11. Procédé selon la revendication 9, dans lequel le solvant à base d'ester organique est l'acétate d'éthyle ou l'acétate d'isopropyle.

12. Procédé selon la revendication 9, dans lequel le solvant à base d'éther organique est l'éther isopropylique ou le méthyl-tertio-butyl-éther.

13. Procédé selon la revendication 1, dans lequel le rapport en poids entre le charbon palladié et le substrat (S)-1-benzyl-3-hydroxy pipéridine de la formule III s ou (R)-1-benzyl-3-hydroxy pipéridine de la formule III r de l'étape 3) est de 0,01 à 0,5:1; la température de réaction est comprise entre 0°C et 80°C; la pression de réaction est de 1,0133 x 10⁵ Pas à 3,04 x 10⁶ Pas (1 atm à 30 atm); dans lequel le rapport molaire entre l'anhydride d'acide t-butoxycarbonylique et le substrat (S)-1-benzyl-3-hydroxy pipéridine de la formule III s ou (R)-1-benzyl-3-hydroxy pipéridine de la formule III r est de 1 à 2:1.
